**Europäisches Patentamt**

**European Patent Office**

**Office européen des brevets**

(11) Veröffentlichungsnummer: **0 149 722**
**B1**

(12) **EUROPÄISCHE PATENTSCHRIFT**

(45) Veröffentlichungstag der Patentschrift:
**23.11.88**

(21) Anmeldenummer: **84111820.1**

(22) Anmeldetag: **03.10.84**

(51) Int. Cl.⁴: **A 61 M 16/00**

(54) **Beatmungssystem.**

(30) Priorität: **20.01.84 DE 3401841**

(43) Veröffentlichungstag der Anmeldung:
**31.07.85 Patentblatt 85/31**

(45) Bekanntmachung des Hinweises auf die Patenterteilung:
**23.11.88 Patentblatt 88/47**

(84) Benannte Vertragsstaaten:
**AT DE FR GB NL SE**

(56) Entgegenhaltungen:
**DE-A-2 543 266**
**DE-A-2 744 327**
**DE-A-2 947 659**
**DE-B-2 505 670**

(73) Patentinhaber: **Drägerwerk Aktiengesellschaft,
Moislinger Allee 53- 55, D-2400 Lübeck 1 (DE)**

(72) Erfinder: **Heim, Ulrich, Dr., Klosterstrasse 19,
D-2067 Reinfeld (DE)**
Erfinder: **Gebhardt, Peter, Dipl.- Phys.,
Gleiwitzstrasse 3, D-2406 Stockelsdorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Beatmungssystem, bei dem eine über ein Atemgasventil von einem Steuergerät gesteuerte Atemgasquelle eine Folge von hochfrequenten Atemgasimpulsen erzeugt, welche einer Jet-Düse im Bereich einer Atemgaszuführungsvorrichtung zugeführt werden, und bei dem das ausgeatmete Gas aus der Atemgaszuführungsvorrichtung über eine Auslaßleitung abgeführt wird, wobie das Steuergerät derart ausgebildet ist, daß nach einer Reihe von Atemgasimpulsen ein Meßinterval eingesteuert wird, in dem von einem Meßsystem Meßwerte an das Steuergerät geführt werden.

Die rasche und genaue Ermittlung von Atemgaswerten und Ventilationsparametern bietet bei der Überwachung von beatmeten Patienten, besonders bei Anwendung des Hochfrequenzimpulsverfahrens mit Jet-Düse (HFJV), wesentliche Schwierigkeiten.

Aus der DE-A-2 947 659 ist bereits ein Beatmungssystem bekannt, wobei die Atemgasquelle bei HFJV-Betrieb Gashochdruckimpulse mit einer Impulsfolgefrequenz > 600/min, erzeugt und bei dem Gasimpulsreihen und Pausen zwischen diesen Impulsreihen vom Steuergerät eingesteuert werden. Diese Pausen sind als Meßabschnitte zur Untersuchung von Gasproben vorgesehen. Die Gasproben werden in einer Gasanalysenvorrichtung analysiert, und nach dem Analysenergebnis kann die Steuerung der Beatmung zur Erreichung eines optimalen Beatmungsprofils durchgeführt werden.

Die endexspiratorische $CO_2$-Konzentration, welche durch die Messung des $CO_2$-Gehaltes der Ausatemluft am Ende einer Exspirationsphase ermittelt wird, ist bei der konventionellen nicht impulsgesteuerten Beatmung (IPPV) zur Bestimmung des $CO_2$-Partialdruckes im Blut allgemein als wichtige Steuergröße anerkannt, Während bei der konventionellen Beatmung eine Bestimmung des $CO_2$-Gehaltes der Ausatemluft keine besonderen technischen Schwierigkeiten mit sich bringt, läßt sich bei der Hochfrequenzbeatmung eine solche $CO_2$-Bestimmung nicht ohne weiteres durchführen, Das ausgeatmete Gas wird durch die kontinuierliche Spülgasströmung stark verdünnt, und das Exspirationsgas weist wegen der geringen Hubvolumina bei der Hochfrequenzbeatmung, die in der Größenordnung des Totraumes der Atemgaszuführungsvorrichtung liegen, nicht die $CO_2$-Konzentration des Alveolarbereichs auf.

Ein weiteres Problem bei der Hochfrequenzbeatmung stellt die Ermittlung des sich in der Lunge einstellenden konstanten Druckuntergrundes, des sogenannten "lungeninternen PEEP" dar. Bei hohen Beatmungsfrequenzen etwa im Bereich von 600/min., wie sie in der Kochfrequenzbeatmung angewendet werden, kommt es durch "airtrapping" zu einem Druckaufbau in der Lunge, der auch am Ende der kurzen Exspirationsphasen nicht abgeklungen ist. Dieser interne PEEP läßt sich mit den bisher bekannten Verfahren nicht hinreichend genau messen. Die bekannte qualitative Messung benutzt die Thoraxblähung, die einen Rückschluß auf den Druckaufbau in der Lunge zuläßt. Hierzu kann die Thorax-Impedanz über am Brustkorb angebrachte Elektroden in bekannter leise bestimmt werden, oder es werden am Brustkorb Dehnungsbänder angebracht, deren Dehnung eine Widerstandsänderung als Meßgröße bewirkt. Diese Meßverfahren liefern hinsichtlich des lungeninternen PEEP nur orientierende Angaben, wobei die Befestigung der entsprechenden Meßelektroden oder Meßbänder, besonders bei Thoraxtraumen, schwierig sein kann.

Die Erfindung geht von der Aufgabenstellung aus, ein Beatmungssystem der eingangs angegebenen Art so auszubilden, daß unter Hochfrequenzbeatmung eine einwandfreie Ermittlung der endexspiratorischen $CO_2$-Konzentration zur Bestimmung des $CO_2$-Partialdrucks im Blut möglich ist und bei dem außerdem der sich bei HFJV in der Lunge aufbauende Druckuntergrund, der lungeninterne PEEP, quantitativ aus der ausgeatmeten Luft bestimmt werden kann. Die Lösung dieser Aufgabenstellung erfolgt dadurch, daß das Steuergerät zusätzlich mit einem vor der Atemgaszuführungsvorrichtung angeordneten Spülgasventil verbunden ist, daß in dem Meßintervall das Atemgasventil und das Spülgasventil geschlossen sind, und daß das Meßsystem strömungsmäßig in der vom Spülgas duchströmten Auslaßleitung angeordnet ist. Atemgasventil und Spülgasventil müssen dabei nicht während des ganzen Meßintervalls gleichzeitig geschlossen sein, weil für bestimmte Anwendungszwecke auch eine phasenverschobene Steuerung dieser Ventile, jedoch in jedem Falle mit einem Überlappungsbereich im Meßintervall, zweckmäßig sein kann.

Weiterbildungen der im Patentanspruch 1 gekennzeichneten Erfindung sind Gegenstand der Patentansprüche 2 - 8. Diese enthalten die spezielle Ausbildung des Meßsystems als $CO_2$-Analysator bzw. als Strömungsmesser sowie Weiterbildungen, bei denen ein Multiplizierer mit dem $CO_2$-Analysator und dem Strömungsmesser verbunden ist, wobei über einen vom Steuergerät gesteuerten Integrator die gesamte abgeatmete $CO_2$-Menge während eines längeren Zeitraums bzw. die $CO_2$-Produktion/Zeiteinheit bestimmt werden können.

Zur Bestimmung der $CO_2$-Konzentration wird in einem solchen Beatmungssystem zunächst durch entsprechende Ansteuerung des Atemgasventils die hochfrequente Beatmung unterbrochen und vor Beginn des Meßintervalls in ein konventionelles Atemmuster überführt. Dies kann zweckmäßig durch entsprechende Verlängerung der Impulsdauer und Verringerung der Pausenintervalle in der Weise erreicht werden, daß der konventionellen Positivüberdruckbeatmung (IPPV) ähnliche Lungenhübe erzeugt werden. Diese dienen dazu, sicherzustellen, daß das im Meßintervall untersuchte Exspirationsgas in seiner $CO_2$-Konzentration der $CO_2$-Konzentration in den Alveolen entspricht. Nach dem letzten durch die Atemgaszuführung ausgelösten Blähhub schaltet das Steuergerät das Atemgasventil und das Spülgasventil in die Schließstellung, so daß in der Auslaßleitung nunmehr Exspirationsgas geführt wird, dessen $CO_2$-Konzentration den alveolären Werten entspricht. Das in die Auslaßleitung eingeschaltete Meßsystem, in diesem Falle ein $CO_2$-Analysator, wird zur Meßwertaufnahme angesteuert.

Zur Bestimmung des lungeninternen PEEP wird die Hochfrequenzbeatmung durch Schließen des Atemgasventils und des Spülgasventils für eine mehreren Hochfrequenzimpulszyklen entsprechende Dauer unterbrochen und gleichzeitig nach dem Ende des letzten Hochfrequenzpulses mit einem Strömungsmesser der Exspirationsflow gemessen und mit einem mit dem Strömungsmesser verbundenen Integrator bis zum Absinken der Strömung auf den Wert Null integriert.

Der Wert des Integrals bis zu dem Zeitpunkt, bei dem bei kontinuierlicher Hochfrequenzimpulsfolge der nächste Hochfrequenzpuls eingesetzt hätte, entspricht dem Tidalvolumen bei der Hochfrequenzbeatmung; das Gesamtintegral abzüglich dem Tidalvolumen entspricht der Untergrundblähung. Bei Kenntnis der Lungencompliance kann aus diesem Wert für die Untergrundblähung durch Division der interne PEEP errechnet werden.

Da sich die Compliance der Lunge während einer Langzeitbeatmung ändern kann, erscheint es zweckmäßig, den Compliancewert kurz vor oder nach der Messung des oben beschriebenen Exspirationsvolumens zu ermitteln. Die Bestimmung des Compliancewertes kann in dem angegebenen Beatmungssystem in Analogie zu dem eingangs angegebenen Meßverfahren zur Bestimmung der endexspiratorischen $CO_2$-Konzentration erfolgen.

Hierzu wird mit der Jet-Düse ein konventioneller Atemhub erzeugt, wobei die Inspirationszeit groß gewählt werden muß, um ein definiertes Druckniveau (Plateau) zu erzeugen. Nach Erreichen dieses vorgegebenen Druckniveaus wird der in den oberen Atemwegen herrschende Druck mit einem in der Atemgaszuführungsvorrichtung distal zur Mündung der Jet-Düse angeordneten Druckaufnehmer gemessen. Während der anschließenden Exspirationsphase werden, wie bei der Bestimmung der endexspiratorischen $CO_2$-Konzentration, die Atemgasströmung zur Jet-Düse mit dem Atemgasventil und die Spülgasströmung mit dem Spülgasventil unterbrochen, so daß über den Strömungsmesser in der Auslaßleitung bei entsprechender Triggerung des Intergrators das Exspirationsvolumen bestimmt werden kann. Aus dem gemessenen Plateaudruck und dem Exspirationsvolumen läßt sich die aktuelle Lungencompliance und damit, wie oben angegeben, auch der lungeninterne PEEP berechnen.

Wesentlich für die erfindungsgemäße Ausbildung des Beatmungssystems ist die Anwendung eines gesteuerten Spülgasventils zusätzlich zu dem die Atemgasimpulse erzeugenden hochfrequent gesteuerten Atemgasventil. Dadurch wird die Möglichkeit geschaffen, unbehindert von der Spülgasströmung, verläßliche Meßwerte über die alveoläre $CO_2$-Konzentration, den lungeninterne PEEP bzw. über die aktuelle Compliance zu erlangen.

In der Zeichnung ist ein Beatmungssystem gemäß der Erfindung in zwei Betriebsarten schematisch dargestellt; es zeigen:

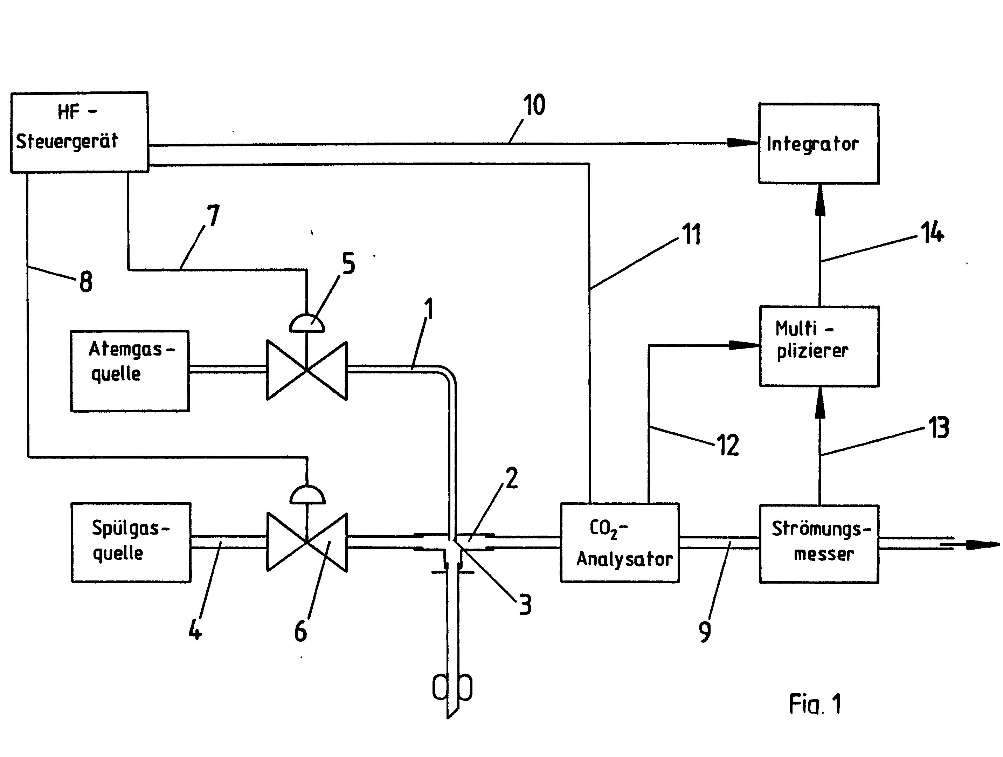

Fig. 1 ein Beatmungssystem mit gesteuertem Atemgas- und Spülgasventil zur Bestimmung der $CO_2$-Konzentration,

Fig. 2 ein Beatmungssystem mit gesteuertem Atemgas- und Spülgasventil in der Schaltung zur Bestimmung des lungeninternen PEEP.

Das Beatmungssystem ist mit einer Atemgasquelle versehen, die über eine Atemgaszuführungsleitung 1 mit einer in der Atemgaszuführungsvorrichtung 2 (T-förmiger Patiententeil) angeordneten Jet-Düse 3 verbunden ist.

Eine Spülgasquelle, die gegebenenfalls mit der Atemgasquelle kombiniert sein kann, ist über eine Spülgasleitung 4 mit der Atemgaszuführungsvorrichtung 2 verbunden. In der Zuführungsleitung 1 ist ein hochfrequent steuerbares Atemgasventil 5 angeordnet. In der Spülgasleitung 4 befindet sich ein steuerbares Spülgasventil 6.

Die Steuerung des Atemgasventils 5 und des Spülgasventils 6 erfolgt über ein HF-Steuergerät, das mit diesen Ventilen über Steuerleitungen 7, 8 verbunden ist.

In der mit der Atemgaszuführungsvorrichtung 2 verbundenen Auslaßleitung 9 sind ein $CO_2$-Analysator und ein Strömungsmesser angeordnet.

Signalleitungen 10, 11 verbinden das HF-Steuergerät mit einem Integrator und mit dem $CO_2$-Analysator. Ausgabeleitungen 12, 13 aus dem $CO_2$-Analysator und dem Strömungsmesser sind an einen Multiplizierer angeschlossen, dessen Ausgang über eine Verbindungsleitung 14 mit dem Integrator verbunden ist.

In diesem Schaltungsaufbau dient das Beatmungssystem mit den beiden steuerbaren Ventilen 5, 6 zur Bestimmung der alveolären $CO_2$-

Konzentration.

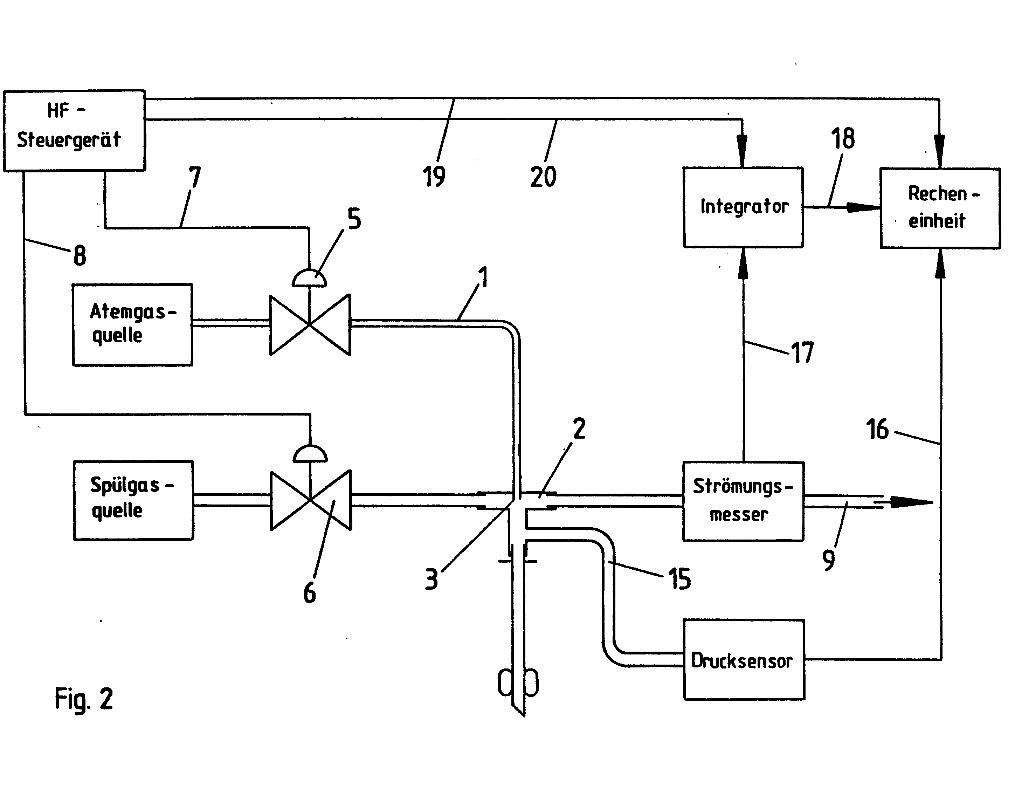

Bei der Betriebsart nach Fig. 2 sind die wesentlichen Teile des Beatmungssystems, nämlich Atemgasquelle, Spülgasquelle, HF-Steuergerät, steuerbare Atemgas- bzw. Spülgasventile sowie Atemgaszuführungsvorrichtung unverändert beibehalten.

In der Atemgaszuführungsvorrichtung 2 befindet sich distal zur Mündung der Jet-Düse 3 die Anschlußleitung 15 eines Drucksensors, der über eine Leitung 16 mit einer Recheneinheit verbunden ist. Der Ausgang des Strömungsmessers führt über eine Ausgangsleitung 17 zum Integrator, welcher durch eine Signalleitung 18 ebenfalls mit der Recheneinheit verbunden ist. Die Recheneinheit steht außerdem über eine weitere Signalleitung 19 mit dem HF-Steuergerät in Verbindung. Eine weitere Signalleitung 20 verbindet das HF-Steuergerät mit dem Integrator.

einem Integrator verbunden ist, der mit dem Steuergerät durch eine Signalleitung (20) in Verbindung steht, und daß in der Atemgaszuführungsvorrichtung (2) ein Drucksensor angeordnet ist, dessen Ausgang mit einer Recheneinheit in Verbindung steht, die über Signalleitungen (18, 19) mit dem Steuergerät und mit dem Integrator verbunden ist.

6. Beatmungssystem nach Anspruch 5, dadurch gekennzeichnet, daß der Drucksensor in der Atemgaszuführungsvorrichtung (2) distal zur Mündung der Jet-Düse (3) angeordnet ist.

7. Beatmungssystem nach Anspruch 1, dadurch gekennzeichnet, daß ein Schaltelement vorgesehen ist, mit dem in einer Reihe von Atemgasimpulsen das von dem Steuergerät eingesteuerte Meßinterval auslösbar ist.

8. Beatmungssystem nach Anspruch 1, dadurch gekennzeichnet, daß das Steuergerät zur Auslösung von Meßintervallen in vorgegebenen Abständen ausgebildet ist.

## Patentansprüche

1. Beatmungssystem, bei dem eine über ein Atemgasventil (5) von einem Steuergerät gesteuerte Atemgasquelle eine Folge von hochfrequenten Atemgasimpulsen erzeugt, welche einer Jet-Düse im Bereich einer Atemgaszuführungsvorrichtung (2) zugeführt werden und bei dem das ausgeatmete Gas aus der Atemgaszuführungsvorrichtung über eine Auslaßleitung (9) abgeführt wird, wobei das Steuergerät derart ausgebildet ist, daß nach einer Reihe von Atemgasimpulsen ein Meßinterval eingesteuert wird, in dem von einem Meßsystem Meßwerte an das Steuergerät geführt werden, dadurch gekennzeichnet, daß das Steuergerät zusätzlich mit einem vor der Atemgaszuführungsvorrichtung (2) liegenden Spülgasventil (6) verbunden ist, daß in dem Meßintervall das Atemgasventil (5) und das Spülgasventil (6) geschlossen sind, und daß das Meßsystem strömungsmäßig in der vom Spülgas durchströmten Auslaßleitung (9) angeordnet ist.

2. Beatmungssystem nach Anspruch 1, dadurch gekennzeichnet, daß in der Auslaßleitung (9) ein $CO_2$-Analysator als Bestandteil des Meßsystems angeordnet ist.

3. Beatmungssystem nach Anspruch 1 oder 2, dadurch gekennzeichnet, daß in der Auslaßleitung (9) ein Strömungsmesser als Bestandteil des Meßsystems angeordnet ist.

4. Beatmungssystem nach Anspruch 3, dadurch gekennzeichnet, daß ein Multiplizierer vorgesehen ist, welcher über Leitungen (12, 13) mit dem $CO_2$-Analysator und dem Strömungsmesser verbunden ist, und daß der Ausgang des Multiplizierers mit einem Integrator verbunden ist, der über eine Signalleitung (10) mit dem Steuergerät in Verbindung steht.

5. Beatmungssystem nach Anspruch 3, dadurch gekennzeichnet, daß der Strömungsmesser mit

## Claims

1. Respiratory system in which a respiratory gas source control led by a control apparatus by way of a respiratory gas valve (5) produces a series of high frequency respiratory gas pulses, which are led to a jet nozzle in the region of a respiratory gas supply device (2) and in which the expired gas is led off from the respiratory gas supply device by way of a discharge line (9), the control apparatus being constructed in such a way that after a series of respiratory gas pulses a measuring interval is guided in, in which measured values are guided to the control apparatus by a measuring system, characterised in that the control apparatus is connected in addition to a cleansing gas valve (6) lying upstream of the respiratory gas supply device (2), in that in the measuring interval the respiratory gas valve (5) and the cleansing gas valve (6) are closed and in that the measuring system is arranged according to current in the discharge line (9) with cleansing gas flowing through.

2. Respiratory system according to claim 1, characterised in that in the discharge line (9) there is arranged a $CO_2$-analyzer as a component part of the measuring system.

3. Respiratory system according to claim 1 or 2, characterised in that in the discharge line (9) there is arranged a current meter as component part of the measuring system.

4. Respiratory system according to claim 3, characterised in that a multiplier is provided which is connected with the $CO_2$-analyzer and the current meter by way of the lines (12,13) and in that the output of the multiplier is connected with an integrator, which is in connection with the control apparatus by way of a signal line (10).

5. Respiratory system according to claim 3, characterised in that the current meter is connected to an integrator which is in connection with the control apparatus by a signal line (20), and in that in the respiratory gas supply device (2) there is arranged a pressure sensor, the output of which is in connection with an arithmetic unit, which is connected to the control apparatus and with the integrator by way of signal lines (18, 19).

6. Respiratory system according to claim 5, characterised in that the pressure sensor is arranged in the respiratory gas supply device (2) distally to the mouth of the jet nozzle (3).

7. Respiratory system according to claim 1, characterised in that a circuit element is provided with which the measuring interval, guided in by the control apparatus, is able to be released in a series of respiratory gas pulses.

8. Respiratory system according to claim 1, characterised in that the control apparatus is constructed for releasing measuring intervals at preset intervals.

**Revendications**

1. Système respiratoire, selon lequel une source de gaz respiratoire asservie par un appareil de commande par l'intermédiaire d'une valve de gaz respiratoire (5) produit une succession d'impulsions de gaz respiratoire à haute fréquence qui sont amenées à une buse à jet dans la région d'un dispositif d'alimentation en gaz respiratoire (2), et selon lequel le gaz expiré du dispositif d'alimentation en gaz respiratoire est évacué par l'intermédiaire d'une conduite d'évacuation (9), l'appareil de commande étant configuré de telle sorte qu'un intervalle de mesure soit introduit à la suite d'une série d'impulsions de gaz respiratoire, pendant lequel des valeurs de mesure sont amenées par un système de mesure à l'appareil de commande, caractérisé en ce que l'appareil de commande est en outre relié à une valve de gaz de rinçage (6) située avant le dispositif d'alimentation en gaz respiratoire (2), en ce que la valve de gaz respiratoire (5) et la valve de gaz de rinçage (6) sont fermées pendant l'intervalle de mesure, et en ce que le système de mesure est disposé dans le flux de la conduite d'évacuation (9) traversée par le gaz de rinçage.

2. Système respiratoire selon la revendication 1, caractérisé en ce qu'un analyseur de $CO_2$ est disposé dans la conduite d'évacuation (9) comme élément du système de mesure.

3. Système respiratoire selon la revendication 1 ou 2, caractérisé en ce qu'un débitmètre est disposé dans la conduite d'évacuation (9) comme élément du système de mesure.

4. Système respiratoire selon la revendication 3, caractérisé en ce qu'il est prevu un multiplicateur, qui est relié à l'analyseur de $CO_2$ et au débitmètre par l'intermédiaire de lignes de transmission (12, 13), et en ce que la sortie du multiplicateur est reliée à un intégrateur qui est lui-même relié à l'appareil de commande par l'intermédiaire d'une ligne de transmission de signaux (10).

5. Système respiratoire selon la revendication 3, caractérisé en ce que le débitmètre est relié à un intégrateur qui est lui-même relié à l'appareil de commande par l'intermédiaire d'une ligne de transmission de signaux (20), et en ce qu'un détecteur de pression est disposé dans le dispositif d'alimentation en gaz respiratoire (2), détecteur dont la sortie est reliée à une unité de calcul qui est elle-même reliée à l'appareil de commande et à l'intégrateur par l'intermédiaire de lignes de transmission de signaux (18, 19).

6. Système respiratoire selon la revendication 5, caractérisé en ce que le détecteur de pression est disposé dans le dispositif d'alimentation en gaz respiratoire (2) de manière distale par rapport à l'embouchure de la buse (3).

7. Système respiratoire selon la revendication 1, caractérisé en ce qu'il est prévu un élément de commutation qui permet de déclencher l'intervalle de mesure introduit par l'appareil de commande dans une série d'impulsions de gaz respiratoire.

8. Système respiratoire selon la revendication 1, caractérisé en ce que l'appareil de commande est conçu pour déclencher des intervalles de mesure à des espacements prédéterminés.

Fig. 1

Fig. 2

0 149 722